# EUROPEAN PATENT SPECIFICATION

(11) **EP 2 742 923 B1**
(45) Date of publication and mention of the grant of the patent: **12.07.2017**
(21) Application number: 12831646.0
(22) Date of filing: 03.09.2012
(51) Int. Cl.: A61H 23/02, A61N 2/02, A45D 33/36, A45D 33/02

(54) **SKIN CARE DEVICE HAVING FUNCTION FOR MOVING VERTICALLY**
HAUTPFLEGEVORRICHTUNG MIT VERTIKALBEWEGUNGSFUNKTION
DISPOSITIF POUR SOIN DE LA PEAU AYANT UNE FONCTION DE DÉPLACEMENT VERTICAL

(30) Priority: 15.09.2011 KR 20110008334 U
(43) Date of publication of application: 18.06.2014
(73) Proprietor: Amorepacific Corporation, Seoul 140-777 (KR)
(72) Inventor: PARK, Wooram, Seoul 140-777 (KR); CHOI, Jungsun, Seoul 140-777 (KR); KIM, Jooho, Seoul 140-777 (KR); HWANG, Yoonkyun, Seoul 140-777 (KR); SHIM, Jongwon, Seoul 140-777 (KR); KIM, Junoh, Seoul 140-777 (KR)
(74) Representative: Eder, Michael
(86) International application number: PCT/KR2012/007033
(87) International publication number: WO 2013/039303

(56) References cited:
- WO-A1-2006/131997
- WO-A2-2011/096660
- KR-B1- 100 602 426
- KR-B1- 100 602 426
- KR-B1- 101 058 359
- KR-U- 20100 008 985
- KR-Y1- 200 399 929
- US-A- 5 361 437

## Description

### [Technical Field]

The present invention relates to a beauty apparatus, and more particularly to a beauty apparatus having a vertical movement function in which a space securing boss is formed on a bottom surface of a guide accommodated in a puff pad such that a space in an interior of the puff pad can be secured even if the puff pad contacts skin of a user to apply a pressure to the skin, thereby improving an in-use feeling of the beauty apparatus.

### [Background Art]

In general, a makeup refers to an action of applying cosmetics to make a user of the cosmetics up pretty, and is used to help appealing portions of the body of the user and correct or camouflage defective portions of the user. The makeup is performed in the order of a sun cream, a makeup base, a primer, a BB cream, powder, and a color makeup after applying base cosmetics.

In particular, a user uses a puff to uniformly apply powder on a surface of his or her skin. The puff includes a product for applying powder by tapping the puff on the skin of a user while being fitted with a finger, and a product for applying powder on skin of the user by automatically vibrating the puff by using a motor.

Document WO2011/096660 describes a beauty apparatus as disclosed in the preamble of claim 1.

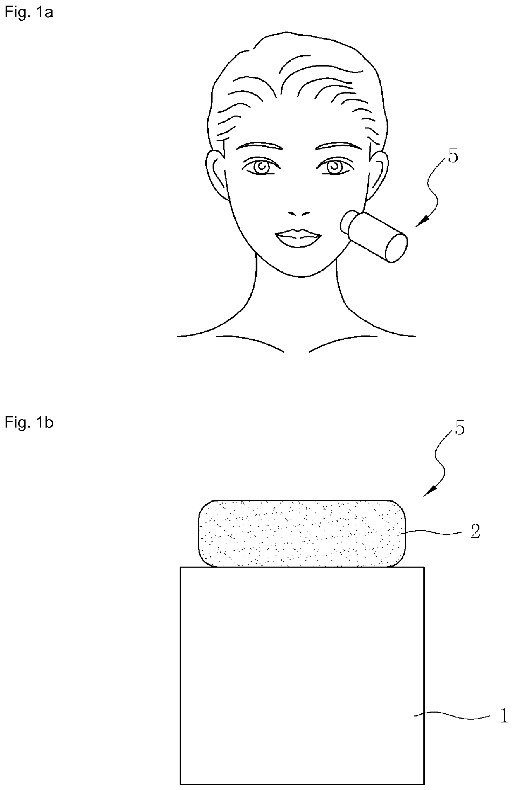

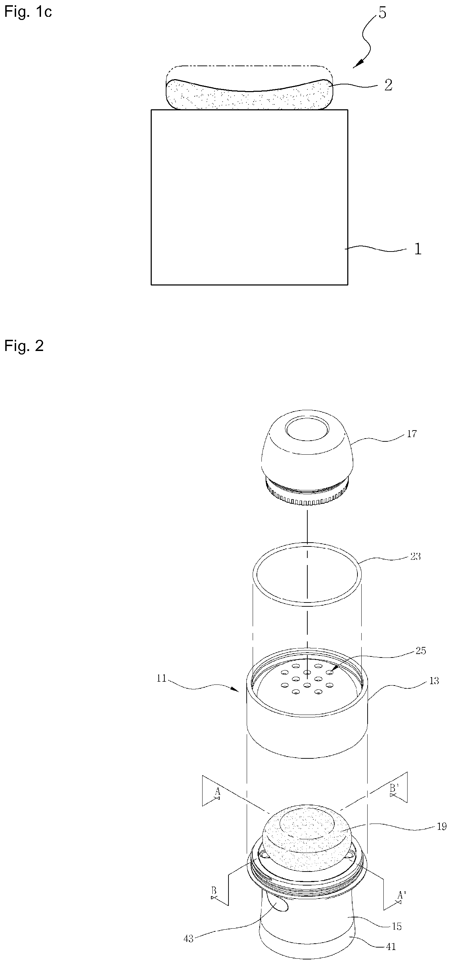

An example of an automatic puff using the motor is shown in FIGS. 1A to 1C. FIGS. 1A to 1C are views for explaining problems of a conventional technology.

As shown in FIGS. 1A to 1C, the conventional automatic puff 5 includes a puff pad 2 mounted to a body 1 in which an electric motor is installed. The automatic puff 5 applies vibrations to the puff pad 2 while an operation shaft connected to the electric motor is rotated by rotating power of the electric motor to apply powder to skin of a user. However, although the conventional automatic puff can conveniently perform a makeup as compared with the manual puff used while being fitted with a finger, powder may not be easily applied when the automatic puff is used in skin having curves, for example, in a face of the user.

Further, in the conventional automatic pad, since a puff pad is mounted to an operation shaft, a movement distance of the puff is long, a speed of tapping skin of a user is slow, and it is difficult to adjust a tapping force. Further, in the conventional automatic puff, an interior space of the puff pad does not exist so that a space in which the operation shaft can move does not exist when a pressure is excessively applied while the automatic puff contacts the skin. Thus, since the conventional automatic puff cannot touch the skin softly, it may cause great inconvenience to the user.

### [Disclosure]

### [Technical Problem]

The present invention has been made in an effort to solve the above-described problems, and it is an object of the present invention to provide a beauty apparatus having a vertical movement function in which a space securing boss is formed on a bottom surface of a guide accommodated in a puff pad such that a minimum space can be secured even in an interior of the puff pad if the puff pad contacts skin of a user to apply a pressure to the skin, thereby improving an in-use feeling of the beauty apparatus.

Another object of the present invention is to provide a beauty apparatus having a vertical movement function that can adjust a frequency of a time-varying magnetic field for vibrating a puff pad to control a striking feeling applied to skin of a user.

Another object of the present invention is to provide a beauty apparatus having a vertical movement function that can prevent a magnetic body coupling part moving according to a time-varying magnetic field within a guide from deviating from the guide.

### [Technical Solution]

In accordance with an aspect of the present invention, there is provided a beauty apparatus including: a case unit including an upper case for accommodating powder and a lower case detachably attached to the upper case; a guide mounted to the lower case between the upper case and the lower case; a magnetic field generating unit mounted to an inside of the lower case between the guide and the lower case, for generating a time-varying magnetic field; a magnetic body coupling part moving through the guide within the lower case according to a change of a magnetic field; a movable plate mounted to the magnetic body coupling part passing through a guide hole formed in the guide on the magnetic body coupling part, for restricting movement of the magnetic body coupling part; and a puff pad enclosing the guide to vibrate according to movement of the movable plate,
wherein the beauty apparatus is characterized in that a space securing boss for maintaining a space in an interior of the puff pad even when the movable plate moves and contacts a bottom surface of the guide is mounted to a bottom surface of the guide,
and wherein the movable plate has a boss through-groove through which the space securing boss passes.

In accordance with an embodiment of the present invention, the beauty apparatus is characterized in that the guide has a flange extending to an inside of the guide hole, for restricting movement of the magnetic body coupling part.

In accordance with an embodiment of the present invention, the beauty apparatus is characterized in that he magnetic body coupling part includes: a movable plate mounting portion passing through the guide hole and to which the movable plate is mounted; a magnetic body positioning portion extending from the movable plate mounting portion and in which a magnetic body for moving the magnetic body coupling part according to a change of a magnetic field is positioned; and a flange contact portion connecting the magnetic body coupling part and the magnetic body positioning portion, for restricting movement of the magnetic body coupling part as the flange contact portion contacts a lower surface of the flange extending to an inside of the guide hole.

In accordance with an embodiment of the present invention, the beauty apparatus is characterized by further including a noise preventing unit formed on a bottom surface of the guide or a lower surface of the movable plate, for removing noise generated due to contact of the guide and the movable plate.

In accordance with an embodiment of the present invention, the beauty apparatus is characterized by further including a noise preventing unit formed on a lower surface of the flange extending to an inside of the guide hole, for restricting movement of the magnetic body coupling part or on the flange contact portion for restricting movement of the magnetic body coupling part as the flange contact portion contacts a lower surface of the flange, for removing noise generated due to contact of the flange and the flange contact portion.

In accordance with an embodiment of the present invention, the beauty apparatus is characterized in that the magnetic field generating unit includes: an AC waveform generating part formed on a circuit board mounted to an inside of the lower case; a coil positioned within the lower case to enclose a magnetic body mounted to the magnetic body coupling part; and a controller formed on the circuit board to control an operation of the AC waveform generating part.

In accordance with an embodiment of the present invention, the beauty apparatus is characterized in that, the magnetic field generating unit is electrically connected to a power source mounted to an inside of the lower case through an on/off operation of a switch mounted to an outside of the lower case.

### [Advantageous Effects]

According to the beauty apparatus having a vertical movement function of the present invention, a space securing boss is formed on a bottom surface of a guide accommodated in a puff pad such that a minimum space can be secured even in an interior of the puff pad if the puff pad contacts skin of a user to apply a pressure to the skin, thereby improving an in-use feeling of the beauty apparatus.

Further, the beauty apparatus having a vertical movement function can adjust a frequency of a time-varying magnetic field for vibrating a puff pad to control a striking feeling applied to skin of a user.

Furthermore, the beauty apparatus having a vertical movement function can prevent a magnetic body coupling part moving according to a time-varying magnetic field within a guide from deviating from the guide.

### [Description of Drawings]

FIGS. 1A to 1C are views for explaining problems of a conventional technology;
FIG. 2 is a perspective view showing a beauty apparatus having a vertical movement function according to an embodiment of the present invention;
FIG. 3 is a partially sectional exploded view showing the beauty apparatus according to the embodiment of the present invention;
FIGS. 4A and 4B are sectional views taken along line A-A' and B-B' of FIG. 2;
FIG. 5 is a block diagram schematically showing an operation circuit applied to the beauty apparatus according to the embodiment of the present invention; and
FIGS. 6A to 6B are views showing an operation of the beauty apparatus having a vertical movement function according to the embodiment of the present invention.

### [Best Mode]

### [Mode for Invention]

The above-described and other objects and new features of the present invention will become clearer with reference to the specification and the accompanying drawings.

Hereinafter, a beauty apparatus having a vertical movement function according to an embodiment of the present invention will be described in detail with reference to FIGS. 2 to 5.

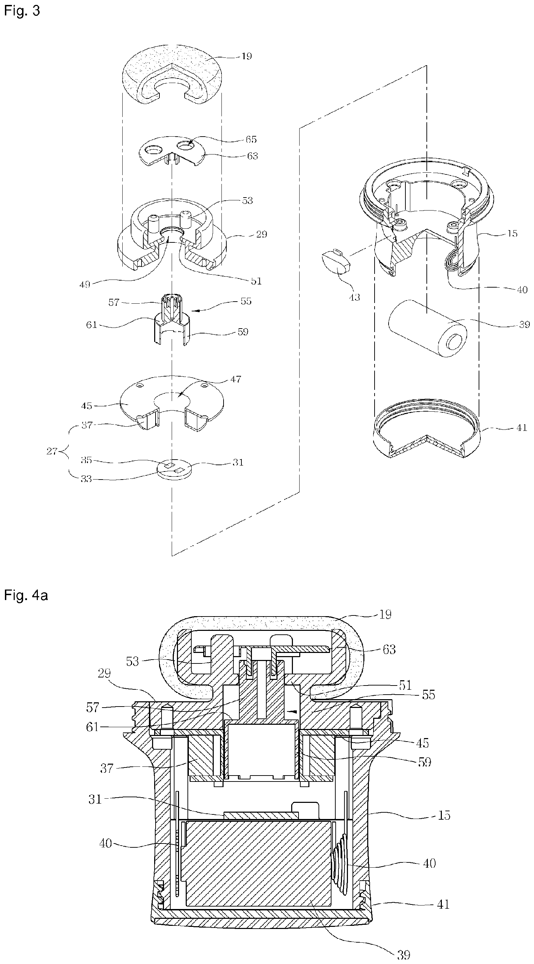

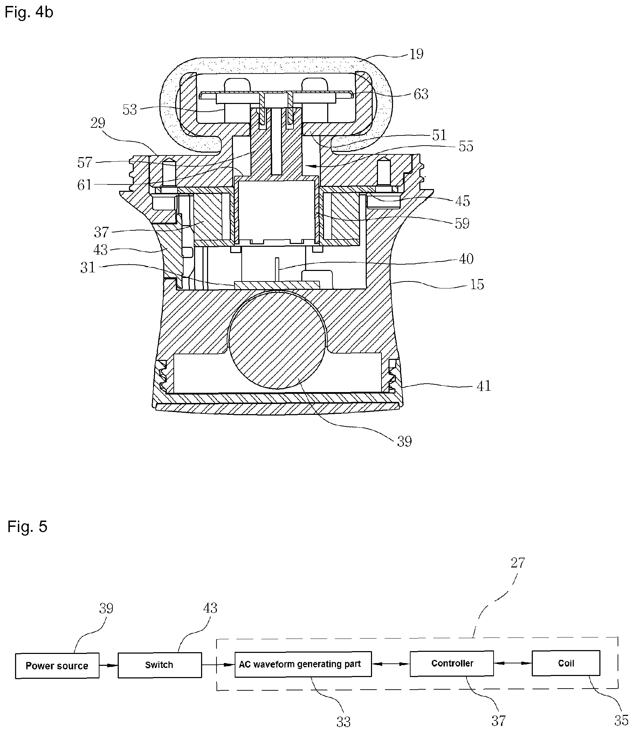

FIG. 2 is a perspective view showing a beauty apparatus having a vertical movement function according to an embodiment of the present invention. FIG. 3 is a partially sectional exploded view showing the beauty apparatus according to the embodiment of the present invention. FIGS. 4A and 4B are sectional views taken along line A-A' and B-B' of FIG. 2. FIG. 5 is a block diagram schematically showing an operation circuit applied to the beauty apparatus according to the embodiment of the present invention.

Referring to FIGS. 2 to 5, the beauty apparatus according to the present invention includes a case unit 11. The case unit 11 includes an upper case 13 for accommodating powder, and a lower case 15 detachably attached to the upper case 13. The upper case 13 and the lower case 15 define an interior space when being coupled to each other. It is preferable that the upper case 13 and the lower case 15 are screw-coupled to each other such that they are attached to or detached from each other.

The upper case 13 includes a powder accommodating portion 17 for accommodating powder, and a pad accommodating portion 21 for accommodating a puff pad 19. The powder accommodating portion 17 is screw-coupled to the pad accommodating portion 21 to be attached to or detached from the pad accommodating portion 21. A packing unit 23 for securely sealing the powder stored in the powder accommodating portion 17, for example, an O-ring may be mounted on an inner wall of the pad accommodating portion 21 to which the powder accommodating portion 17 is mounted. The pad accommodating portion 21 includes a powder moving hole 25 for supplying powder to the puff pad 19.

Meanwhile, a magnetic field generating unit 27 and a guide 29 are sequentially located from a bottom surface of the lower case 15 between the upper case 13 and the lower case 15. The magnetic field generating unit 27 generates a time-varying magnetic field. The magnetic field generating unit 27 includes an AC waveform generating part 33 formed on a circuit board 31 mounted to an inside of the lower case 15, a coil 35, and a controller 37. The circuit board 31 is electrically connected to a power source 39 mounted to a lower portion of the lower case 15 by an electrode contact piece 40. In this case, the power source 39 may include a DC power source, for example, a primary battery or a secondary battery which is commercially available, and be easily replaced through attaching and detaching a lower cap 41 which is detachably attached to a lower portion of the lower case 15 through screw-coupling.

The AC waveform generating part 33 is electrically connected to the power source 39 through a switch 43 passing through the lower case 15. The AC waveform generating part 33 may convert a DC voltage input from the power source 39 to an AC voltage and supplies the AC voltage to the coil 35. For example, the AC waveform generating part 33 generates a pulse by using a timer IC, determines a frequency and a duty ratio of the puff pad 19 by using a time constant, and may apply an AC voltage to the coil 35 by applying an H-bridge circuit including four transistors. In this case, the switch 43 may be a tack switch that is operated only while being pressed and interrupts flow of a current applied to the AC waveform generating part 33 when being switched off.

The coil 35 is wound in a cylindrical coil accommodating part 45 mounted to the lower case 15, and the coil accommodating part 45 has a magnetic body through-hole 47 in which a magnetic body is located. An attractive force and a repulsive force are applied to the magnetic body according to a change of a magnetic field generated by the coil 35 such that the magnetic body moves in the magnetic body through-hole 47. The controller 37 controls an operation of the AC waveform generating part 33 formed on the circuit board 31 to determine a frequency of the puff pad 19.

Meanwhile, the guide 29 is mounted inside the lower case 15 and in the upper part of the coil accommodating part 45. The guide 29 includes a guide hole 49, a flange 51, and a space securing boss 53. A magnetic body coupling part 55 to which the magnetic body is coupled such that the magnetic body moves according to a change of a magnetic field generated by the coil 35 is located in the guide hole 49. In this case, it is preferable that the magnetic body coupling part 55 moves within the magnetic body through-hole 47 of the coil accommodating part 45 and the guide hole 49 of the guide 29.

The flange 51 extends to an inside of the guide hole 49 to restrict movement of the magnetic body coupling part 55. That is, the flange 51 may prevent the moving magnetic body coupling part 55 from escaping from the guide 29. The space securing boss 53 protrudes from a bottom surface of the guide 29 within the puff pad 19. It is preferable that a plurality of space securing bosses 53 are provided. When the puff pad 19 contacts skin of a user to be pressed, the space securing boss 53 maintains a predetermined space in the puff pad 19 such that the puff pad 19 transfers vibrations constantly along a curve of the skin. Thus, the space securing boss 53 improves an in-use feeling of the puff pad 19 through generating vibrations of the puff pad 19 while making tight contact with skin.

The magnetic body coupling part 55 moves along the magnetic body through-hole 47 and the guide hole 49 of the guide 29 according to a change of a magnetic field generated by the coil 35. The magnetic body coupling part 55 includes a movable plate mounting portion 57 passing through the guide hole 49, a magnetic body positioning portion 59, and a flange contact portion 61. The movable plate mounting portion 57 moves through the guide hole 49, and is coupled to a movable plate 63 that moves together with the magnetic body coupling part 55 on the guide 29. In this case, the movable plate 63 includes a boss through-hole 65 through which the space securing boss 53 passes.

The magnetic body positioning portion 59 extends from the movable plate mounting portion 57 and is located within the magnetic body through-hole 47. A magnetic body, for example, a magnet is mounted to the magnetic body positioning portion 59, and the magnet moves the magnetic body coupling part 55 according to a change in a magnetic field generated by the coil 35. It is preferable that the magnetic body positioning portion 59 is wider than the movable plate mounting portion 57. The flange contact portion 61 connects the movable plate mounting portion 57 and the magnetic body positioning portion 59, and contacts a lower surface of the flange 15 to restrict movement of the magnetic body coupling part 55 when the magnetic body coupling part 55 moves. Thus, the flange 51 and the movable plate 63 restrict a linear movement length of the guide 29 to adjust vibrations applied to the puff pad 19 by the movable plate 63.

In this case, a noise preventing unit (not shown) formed on a bottom surface of the guide 29 or a lower surface of the movable plate 63 to remove noise generated due to contact of the guide 29 and the movable plate 63 may be provided. For example, it is preferable that the noise preventing unit is formed of a material having a resilient force, such as sponge or rubber. Further, the noise preventing unit may be formed on a lower surface of the flange 51 or the flange contact portion 61 to remove noise generated due to contact of the flange 51 and the flange contact portion 61.

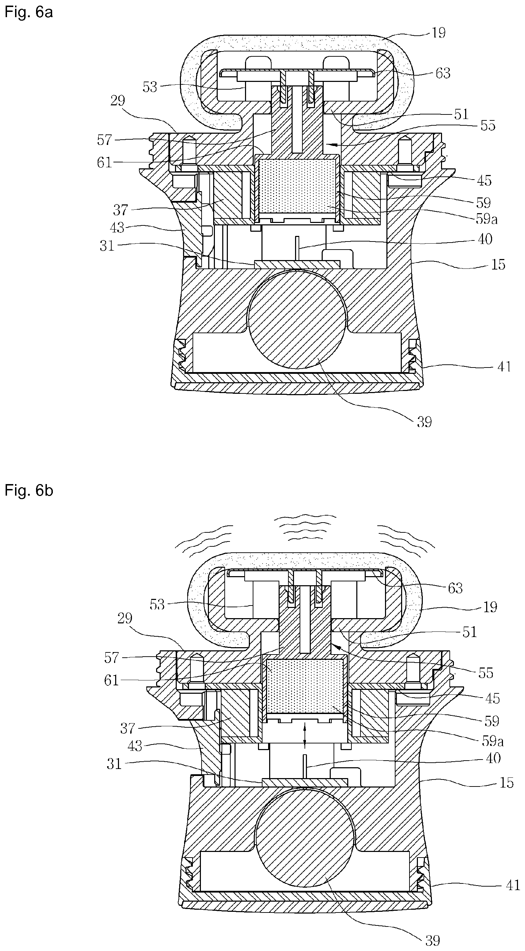

Next, an operation of the beauty apparatus having a vertical movement function according to the embodiment of the present invention will be described with reference to FIGS. 4B, 6A, and 6B.

FIGS. 6A and 6B are views showing an operation of the beauty apparatus having a vertical movement function according to the embodiment of the present invention.

Referring to FIGS. 4B, 6A, and 6B, since the beauty apparatus according to the present invention does not generate a magnetic field in the coil as a current is not applied to the AC waveform generating part 33 when the switch 43 is off, vibrations are not applied to the puff pad 19. However, when a user continuously maintains the switch 43 in ON state, a current is applied to the AC waveform generating part 33 such that an AC voltage is generated by the DC power source 39.

The AC voltage generates a time-varying magnetic field in the coil 35, and an attractive force and a repulsive force are alternately applied to a magnet 59a attached to the magnetic body positioning portion 59 by the time-varying magnetic field. The magnetic body coupling part 55 to which the magnet 59a is attached reciprocates between the magnetic body through-hole 47 and the guide hole 49, and the movable plate 63 that moves together with the magnetic body coupling part 55 generates vibrations of the puff pad 19 within the puff pad 19.

In this case, the beauty apparatus secures a minimum space in the puff pad 19 by using the space securing boss 53 attached to the guide 29 to improve an in-user feeling of the puff pad 19 attached to skin of the user.

Although the exemplary embodiment of the present invention has been described in detail, the present invention is not limited by the embodiment.

### [Description of Reference Numerals]

15: Lower case 19: Puff pad
27: Magnetic field generating unit 29: Guide
33: AC waveform generating part 35: Coil
39: Power source 43: Switch
45: Coil accommodating part 47: Magnetic body through-hole
49: Guide hole 51: Flange
53: Space securing boss 55: Magnetic body coupling part
57: Movable plate mounting portion 59: Magnetic body positioning portion
59a: Magnet 61: Flange contact portion
63: Movable plate 65: Boss through-hole

## Claims

1. A beauty apparatus comprising:
a case unit (11) comprising an upper case (13) for accommodating powder and a lower case (15) detachably attached to the upper case (13);
a guide (29) mounted to the lower case (15) between the upper case (13) and the lower case (15);
a magnetic field generating unit (27) mounted to an inside of the lower case (15) between the guide (29) and the lower case (15), for generating a time-varying magnetic field;
a magnetic body coupling part (55) moving through the guide (29) within the lower case (15) according to a change of a magnetic field;
a movable plate (63) mounted to the magnetic body coupling part (55) passing through a guide hole (49) formed in the guide (29), for restricting movement of the magnetic body coupling part (55); and
a puff pad (19) enclosing the guide (29) to vibrate according to movement of the movable plate (63),
**characterized in that** a space securing boss (53) protrudes from a bottom surface of the guide (29) within the puff pad (19) for maintaining a space in an interior of the puff pad (19) even when the movable plate (63) moves and contacts a bottom surface of the guide (29), and **in that** the movable plate (63) has a boss through-groove (65) through which the space securing boss (53) passes.

2. The beauty apparatus of claim 1, wherein the guide (29) has a flange (51) extending to an inside of the guide hole (49), for restricting movement of the magnetic body coupling part (55).

3. The beauty apparatus of claim 1 or 2, wherein the magnetic body coupling part (55) comprises:
a movable plate mounting portion (57) passing through the guide hole (49) and to which the movable plate (63) is mounted;
a magnetic body positioning portion (59) extending from the movable plate mounting portion 57 and in which a magnetic body for moving the magnetic body coupling part (55) according to a change of a magnetic field is positioned; and
a flange contact portion (61) connecting the magnetic body coupling part (55) and the magnetic body positioning portion (59), for restricting movement of the magnetic body coupling part (55) as the flange contact portion (61) contacts a lower surface of the flange (51) extending to an inside of the guide hole (49).

4. The beauty apparatus of claim 1 or 2, further comprising a noise preventing unit formed of a material having a resilient force on a bottom surface of the guide (29) or a lower surface of the movable plate (63), for removing noise generated due to contact of the guide (29) and the movable plate (63).

5. The beauty apparatus of claim 1 or 2, further comprising a noise preventing unit formed on a lower surface of the flange (51) extending to an inside of the guide hole (49), for restricting movement of the magnetic body coupling part (55) or on the flange contact portion (61) for restricting movement of the magnetic body coupling part (55) as the flange contact portion (61) contacts a lower surface of the flange (51), for removing noise generated due to contact of the flange (51) and the flange contact portion (61).

6. The beauty apparatus of claim 1, wherein the magnetic field generating unit 27 comprises:
an AC waveform generating part (33) formed on a circuit board (31) mounted to an inside of the lower case (15), wherein the AC waveform generating part (33) is capable of converting a DC voltage input from a power source (39) to an AC voltage, and supplies the AC voltage to a coil (35);
a coil (35) positioned within the lower case (15) to enclose a magnetic body mounted to the magnetic body coupling part (55); and
a controller (37) formed on the circuit board (31) to control an operation of the AC waveform generating part (33).

7. The beauty apparatus of claim 6, wherein the magnetic field generating unit (27) is electrically connected to a power source (39) mounted to an inside of the lower case (15) through an on/off operation of a switch (43) mounted to an outside of the lower case (15).

## Patentansprüche

1. Schönheitsapparat, umfassend:
eine Gehäuseeinheit (11), umfassend ein oberes Gehäuse (13) zum Aufnehmen von Puder und ein am oberen Gehäuse (13) entfernbar befestigtes, unteres Gehäuse (15);
eine an dem unteren Gehäuse (15) zwischen dem oberen Gehäuse (13) und dem unteren Gehäuse (15) montierte Führung (29);
eine magnetfelderzeugende Einheit (27), montiert an eine Innenseite des unteren Gehäuses (15) zwischen der Führung (29) und dem unteren Gehäuse (15), zum Erzeugen eines zeitlich veränderlichen Magnetfelds;
ein sich gemäß einer Änderung eines Magnetfelds durch die Führung (29) hindurch bewegendes Magnetkörper-Kopplungsteil (55) innerhalb des unteren Gehäuses (15);
eine auf das Magnetkörper-Kopplungsteil (55) montierte, bewegliche Platte (63) zum Einschränken der Bewegung des Magnetkörper-Kopplungsteils (55), die durch ein in der Führung (29) gebildetes Führungsloch (49) verläuft; und
ein die Führung (29) umschließendes Quasten-Pad (19) zum Vibrieren gemäß der Bewegung der beweglichen Platte (63),
**gekennzeichnet durch** einen raumsichernden Vorsprung (53) aus einer Bodenoberfläche der Führung (29) innerhalb des Quasten-Pads (19) zum Aufrechterhalten eines Raums in einem Inneren des Quasten-Pads (19) hinausragt, auch wenn die bewegliche Platte (63) sich bewegt und eine Bodenoberfläche der Führung (29) berührt, und wobei die bewegliche Platte (63) eine Vorsprungs-Durchgangsnut (65) aufweist, durch welche der raumsichernde Vorsprung (53) hindurchgeht.

2. Schönheitsapparat nach Anspruch 1, wobei die Führung (29) einen Flansch (51) zum Einschränken der Bewegung des Magnetkörper-Kopplungsteils (55) aufweist, der sich zu einer Innenseide des Führungslochs (49) erstreckt.

3. Schönheitsapparat nach Anspruch 1 oder 2, wobei das Magnetkörper-Kopplungsteil (55) Folgendes umfasst:
einen Montierungsabschnitt (57) einer beweglichen Platte, der durch das Führungsloch (49) verläuft, und an welchen die bewegliche Platte (63) montiert ist;
einen Magnetkörper-Positionierungsabschnitt (59), der sich von dem Montierungsabschnitt (57) der beweglichen Platte aus erstreckt, und in welchem ein Magnetkörper zum Bewegen des Magnetkörper-Kopplungsteils (55) gemäß einer Änderung eines Magnetfeldes positioniert ist; und
einen Flanschkontaktabschnitt (61), der das Magnetkörper-Kopplungsteil (55) und den Magnetkörper-Positionierungsabschnitt (59) zum Einschränken der Bewegung des Magnetkörper-Kopplungsteils (55) verbindet, wenn der Flanschkontaktabschnitt (61) eine untere Oberfläche des Flansches (51), der sich zu einer Innenseite des Führungslochs (49) erstreckt, berührt.

4. Schönheitsapparat nach Anspruch 1 oder 2, ferner umfassend eine lärmvermeidende Einheit, gebildet aus einem Material mit einer Federkraft auf einer Bodenoberfläche der Führung (29) oder einer unteren Oberfläche der beweglichen Platte (63) zum Entfernen von durch den Kontakt der Führung (29) und der beweglichen Platte (63) erzeugten Lärm.

5. Schönheitsapparat nach Anspruch 1 oder 2, ferner umfassend eine auf einer unteren Oberfläche des Flansches (51), der sich zu einer Innenseite des Führungslochs (49) erstreckt, zum Einschränken der Bewegung des Magnetkörper-Kopplungsteils (55), oder auf dem Flanschkontaktabschnitt (61) zum Einschränken der Bewegung des Magnetkörper-Kopplungsteils (55), wenn der Flanschkontaktabschnitt (61) die untere Oberfläche des Flansches (51) berührt, gebildete, lärmvermeidende Einheit zum Entfernen des Lärms, der durch den Kontakt des Flansches (51) und des Flanschkontaktabschnitts (61) erzeugt wird.

6. Schönheitsapparat nach Anspruch 1, wobei die magnetfelderzeugende Einheit (27) Folgendes umfasst:
einen AC-Wellenform erzeugenden Teil (33), gebildet auf einer an die Innenseite des unteren Gehäuses (15) montierte Leiterplatte (31),
wobei der AC-Wellenform erzeugende Teil (33) in der Lage ist, eine DC-Spannungseingabe von einer Stromquelle (39) zu einer AC-Spannung umzuwandeln, und die AC-Spannung an eine Spule (35) liefert;
eine innerhalb des unteren Gehäuses (15) positionierte Spule (35), um einen an den Magnetkörper-Kopplungsteil (55) montierten Magnetkörper zu umschließen; und
eine auf der Leiterplatte (31) gebildete Steuerung (37), um einen Betrieb des AC-Wellenform erzeugenden Teils (33) zu steuern.

7. Schönheitsapparat nach Anspruch 6, wobei die magnetfelderzeugende Einheit (27) durch einen Ein/Aus-Betrieb eines an eine Außenseite des unteren Gehäuses (15) montierten Schalters (43) elektrisch mit einer an eine Innenseite des unteren Gehäuses (15) montierten Stromquelle (39) verbunden ist.

## Revendications

1. Appareil d'esthétique, comprenant :
une unité boîtier (11) comprenant un boîtier supérieur (13) pour loger de la poudre et un boîtier inférieur (15) fixé de manière détachable au boîtier supérieur (13) ;
un guide (29) monté sur le boîtier inférieur (15) entre le boîtier supérieur (13) et le boîtier inférieur (15) ;
une unité de production de champ magnétique (27) montée sur un intérieur du boîtier inférieur (15) entre le guide (29) et le boîtier inférieur (15), afin de produire un champ magnétique variable dans le temps ;
une partie de couplage de corps magnétique (55) se déplaçant à travers le guide (29) au sein du boîtier inférieur (15) en fonction d'un changement d'un champ magnétique ;
une plaque mobile (63) montée sur la partie de couplage de corps magnétique (55) passant à travers un trou de guidage (49) formé dans le guide (29) afin de restreindre le mouvement de la partie de couplage de corps magnétique (55) ; et
un tampon de houppette (19) enserrant le guide (29) afin de vibrer en fonction du mouvement de la plaque mobile (63),
**caractérisé par** un bossage de garantie d'espace (53) fait saillie depuis une surface inférieure du guide (29) au sein du tampon de houppette (19) afin de maintenir un espace dans un intérieur du tampon de houppette (19) même lorsque la plaque mobile (63) se déplace et entrer en contact avec une surface inférieure du guide (29) et dans lequel la plaque mobile (63) présente une rainure traversante de bossage (65) à travers laquelle le bossage de garantie d'espace (53) passe.

2. Appareil d'esthétique selon la revendication 1, dans lequel le guide (29) présente un rebord (51) s'étendant jusqu'à un intérieur du trou de guidage (49), pour restreindre le mouvement de la partie de couplage de corps magnétique (55).

3. Appareil d'esthétique selon la revendication 1 ou 2, dans lequel la partie de couplage de corps magnétique (55) comprend :
une portion de montage de plaque mobile (57) passant à travers le trou de guidage (49) et sur laquelle la plaque mobile (63) est montée ;
une portion de positionnement de corps magnétique (59) s'étendant depuis la portion de montage de plaque mobile (57) et dans laquelle un corps magnétique pour déplacer la partie de couplage de corps magnétique (55) en fonction d'un changement d'un champ magnétique est positionné ; et
une portion de contact de rebord (61) reliant la partie de couplage de corps magnétique (55) et la portion de positionnement de corps magnétique (59), pour restreindre le mouvement de la partie de couplage de corps magnétique (55) lorsque la portion de contact de rebord (61) entre en contact avec une surface inférieure du rebord (51) s'étendant jusqu'à un intérieur du trou de guide (49).

4. Appareil d'esthétique selon la revendication 1 ou 2, comprenant en outre une unité de prévention du bruit formée d'un matériau ayant une force élastique sur une surface inférieure du guide (29) ou une surface inférieure de la plaque mobile (63), afin de supprimer le bruit provoqué par le contact entre le guide (29) et la plaque mobile (63).

5. Appareil d'esthétique selon la revendication 1 ou 2, comprenant en outre une unité de prévention du bruit formée sur une surface inférieure du rebord (51) s'étendant jusqu'à un intérieur du trou de guidage (49), afin de restreindre le mouvement de la partie de couplage de corps magnétique (55) ou sur la portion de contact de rebord (61) pour restreindre le mouvement de la partie de couplage de corps magnétique (55) lorsque la portion de contact de rebord (61) est en contact avec une surface inférieure du rebord (51), afin de supprimer le bruit provoqué par le contact entre le rebord (51) et la portion de contact de rebord (61).

6. Appareil d'esthétique selon la revendication 1, dans lequel l'unité de production de champ magnétique (27) comprend :
une partie de production de forme d'onde C.A. (33) formée sur une carte de circuit (31) montée sur un intérieur du boîtier inférieur (15), dans lequel la partie de production de forme d'onde C.A. (33) est apte à convertir une entrée de tension C.C. provenant d'une source d'alimentation (39) en une tension C.A. et fournit la tension C.A. à une bobine (35) ;
une bobine (35) positionnée à l'intérieur du boîtier inférieur (15) afin d'enserrer un corps magnétique monté sur la partie de couplage de corps magnétique (55) ; et
un contrôleur (37) formé sur la carte de circuit (31) afin de contrôler un fonctionnement de la partie de production de forme d'onde C.A..

7. Appareil d'esthétique selon la revendication 6, dans lequel l'unité de production de champ magnétique (27) est électriquement connectée à une source d'alimentation (39) montée sur un intérieur du boîtier inférieur (15) à travers un actionnement marche/arrêt d'un commutateur (43) monté sur un extérieur du boîtier inférieur (15).
